# EUROPEAN PATENT APPLICATION

(11) **EP 4 790 414 A1**
(43) Date of publication of application: **12.08.2026**
(21) Application number: 23954740.9
(22) Date of filing: 04.10.2023
(51) Int. Cl.: G01N 33/48, G01N 33/483

(54) **PATHOLOGICAL DIAGNOSIS SUPPORT DEVICE, PATHOLOGICAL DIAGNOSIS SUPPORT METHOD, AND PROGRAM**

(71) Applicant: NEC CORPORATION, Minato-ku, Tokyo 108-8001 (JP)
(72) Inventor: OMI, Yasuo, Tokyo 108-8001 (JP)
(74) Representative: Betten & Resch
(86) International application number: PCT/JP2023/036153
(87) International publication number: WO 2025/074528

(57) **Abstract**

The present invention provides a pathological diagnosis support apparatus that comprises: a determination unit that determines whether or not correction is necessary to optimize a classification result in intraoperative rapid cytodiagnosis, said correction being correction of a parameter indicating an effective magnification, which is the magnification of the size of a subject in an image of interest relative to the actual size of the subject, the image of interest having been obtained by using a camera to capture a microscope-produced image of a specimen cell to be classified as benign or malignant in intraoperative rapid cytodiagnosis; a notification unit that issues a notification indicating that parameter correction is necessary if correction is determined to be necessary; and a classification unit that classifies the specimen cell as benign or malignant if correction is determined to be unnecessary.

## Description

### Technical Field

The present disclosure relates to a pathological diagnosis support apparatus, a pathological diagnosis support method, and a program.

### Background Art

A technique related to benign/malignant classification for classifying whether cells included in an image as a subject are benign or malignant has been known.

For example, PTL 1 discloses a classification device that classifies whether cells included, as subjects, in an image including a partial range of a pathological sample as the subject are benign or malignant. The classification device adjusts an imaging magnification of the image, according to the number of cells included in the image as the subject.

### Citation List

### Patent Literature

PTL 1: WO 2023-105548 A1

### Summary of Invention

### Technical Problem

In AI diagnosis support in Rapid On-Site Evaluation (ROSE), an image including a specimen cell group including at least one specimen cell to be a benign/malignant classification target, as a subject, is captured by using a camera installed in a microscope.

With this configuration, even in an image obtained by imaging the same subject, there is a possibility that sizes of the subject in the images are different. For example, in a case where the same subject is imaged using two different microscopes, even if the two different microscopes image the same pathological sample using the same camera and an objective lens having the same nominal value, since a distance from a focal point of the camera to the subject may be different, there is a possibility that the sizes of the subject in the images are different.

In a plurality of images imaged using the same microscope, the same camera, and the same objective lens, there is a possibility that the distance from the focal point of the camera to the subject changes with time or due to a physical impact, while using the microscope. Therefore, even in a case where the same subject is imaged using the same microscope, the same camera, and the same objective lens, there is a possibility that the sizes of the subject in the images are different.

In this way, even for the same subject, there is a case where the sizes in the image are different, between the plurality of images. That is, even if the subject is imaged using the objective lens having the same nominal value, in the microscope in which the camera is installed, there is a case where a magnification (effective magnification) of an actually captured image is different. If benign/malignant classification is performed on a specimen cell group using the plurality of images imaged at the different effective magnifications, there is a possibility that accuracy in the benign/malignant classification is lowered.

The present disclosure has been made in view of the above problems, and an exemplary object of the present disclosure is to provide a technology for improving accuracy in benign/malignant classification on a specimen cell group in pathological diagnosis.

### Solution to Problem

A pathological diagnosis support apparatus according to one exemplary aspect of the present disclosure includes acquisition means for acquiring a target image obtained by capturing, with a camera, an image in which a specimen cell to be classified as benign or malignant in rapid on-site evaluation is projected with a microscope and a parameter indicating an effective magnification that is a magnification of a size of a subject in the target image, with respect to an actual subject size, determination means for determining whether it is necessary to correct the parameter, notification means for notifying that it is necessary to correct the parameter, in a case where the determination means determines that the correction is necessary, and classification means for performing benign/malignant classification on the specimen cell, using the parameter, in a case where the determination means determines that the correction is not necessary.

A pathological diagnosis support method according to one exemplary aspect of the present disclosure performed by at least one processor, includes acquisition processing for acquiring a target image obtained by capturing, with a camera, an image in which a specimen cell to be classified as benign or malignant in rapid on-site evaluation is projected with a microscope and a parameter indicating an effective magnification that is a magnification of a size of a subject in the target image, with respect to an actual subject size, determination processing for determining whether it is necessary to correct the parameter, notification processing for notifying that it is necessary to correct the parameter, in a case where it is determined in the determination processing that the correction is necessary, and classification processing for performing benign/malignant classification on the specimen cell, using the parameter, in a case where it is determined in the determination processing that the correction is not necessary.

A program according to one exemplary aspect of the present disclosure for causing a computer to function as a pathological diagnosis support apparatus, the program causes the computer to execute acquisition processing for acquiring a target image obtained by capturing, with a camera, an image in which a specimen cell to be classified as benign or malignant in rapid on-site evaluation is projected with a microscope and a parameter indicating an effective magnification that is a magnification of a size of a subject in the target image, with respect to an actual subject size, determination processing for determining whether it is necessary to correct the parameter, notification processing for notifying that it is necessary to correct the parameter, in a case where it is determined in the determination processing that the correction is necessary, and classification processing for performing benign/malignant classification on the specimen cell, using the parameter, in a case where it is determined in the determination processing that the correction is not necessary.

### Advantageous Effects of Invention

According to one exemplary aspect of the present disclosure, an exemplary effect is obtained that it is possible to provide a technology for improving accuracy in benign/malignant classification on a specimen cell, in pathological diagnosis.

### Brief Description of Drawings

[Fig. 1] Fig. 1 is a block diagram illustrating a configuration of a pathological diagnosis support apparatus according to the present disclosure.
[Fig. 2] Fig. 2 is a flowchart illustrating a flow of a pathological diagnosis support method according to the present disclosure.
[Fig. 3] Fig. 3 is a diagram illustrating an example of a state of pathological diagnosis using the pathological diagnosis support apparatus according to the present disclosure.
[Fig. 4] Fig. 4 is a block diagram illustrating the configuration of the pathological diagnosis support apparatus according to the present disclosure.
[Fig. 5] Fig. 5 is a flowchart illustrating the flow of the pathological diagnosis support method according to the present disclosure.
[Fig. 6] Fig. 6 is a flowchart illustrating a flow of processing executed by a determination unit according to the present disclosure.
[Fig. 7] Fig. 7 is a diagram illustrating an example of processing for calculating a similarity by a calculation unit according to the present disclosure.
[Fig. 8] Fig. 8 is flowchart illustrating a flow of processing executed by a correction unit according to the present disclosure.
[Fig. 9] Fig. 9 is an example of an image processed by the correction unit according to the present disclosure.
[Fig. 10] Fig. 10 is a block diagram illustrating a configuration of a computer that functions as the pathological diagnosis support apparatus according to the present disclosure.

### Example Embodiment

Hereinafter, example embodiments of the present invention will be described. However, the present invention is not limited to the following exemplary example embodiments, and various modifications can be made within a scope described in the claims. For example, example embodiments obtained by appropriately combining technical means adopted in each of the exemplary example embodiments described below can also be included in the scope of the present invention. Example embodiments obtained by appropriately omitting some of the technical means adopted in each of the exemplary example embodiments described below can also be included in the scope of the present invention. Effects mentioned in each of the following exemplary example embodiments are examples of effects expected in the exemplary example embodiment, and do not define extension of the present invention. That is, example embodiments that do not achieve the effects mentioned in each of the exemplary example embodiments described below can also be included in the scope of the present invention.

### [First Exemplary Example Embodiment]

A first exemplary example embodiment that is an example of the example embodiments of the present invention will be described in detail with reference to the drawings. The present exemplary example embodiment is a basic form of each exemplary example embodiment described below. An application range of each technical means adopted in the present exemplary example embodiment is not limited to the present exemplary example embodiment. That is, each technical means adopted in the present exemplary example embodiment can also be adopted in another exemplary example embodiment included in the present disclosure as long as no particular technical problem occurs. Each technical means illustrated in the drawings referred to for describing the present exemplary example embodiment can also be adopted in another exemplary example embodiment included in the present disclosure as long as no particular technical problem occurs.

### (Configuration of Pathological diagnosis support apparatus)

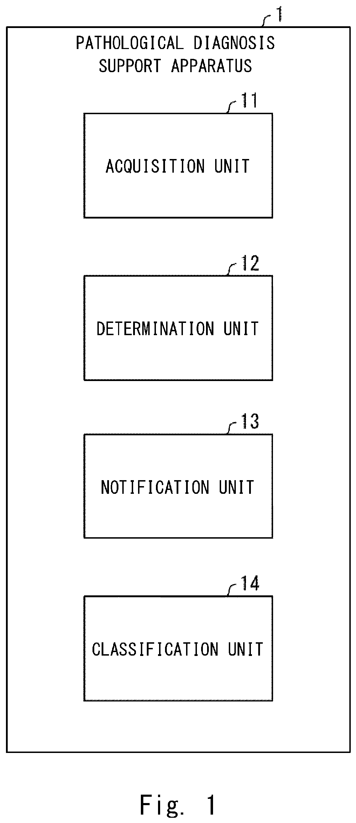

A configuration of a pathological diagnosis support apparatus 1 will be described with reference to Fig. 1. Fig. 1 is a block diagram illustrating the configuration of the pathological diagnosis support apparatus 1. As illustrated in Fig. 1, the pathological diagnosis support apparatus 1 includes an acquisition unit 11, a determination unit 12, a notification unit 13, and a classification unit 14. The acquisition unit 11, the determination unit 12, the notification unit 13, and the classification unit 14 are respectively configurations for achieving acquisition means, determination means, notification means, and classification means, in the present exemplary example embodiment.

The acquisition unit 11 acquires a target image obtained by capturing, with a camera, an image in which a specimen cell to be classified as benign or malignant in rapid on-site evaluation is projected with a microscope and a parameter indicating an effective magnification that is a magnification of a size of a subject in the target image, with respect to an actual subject size. The acquisition unit 11 supplies the acquired target image to the classification unit 14 and supplies the acquired parameter to the determination unit 12.

The acquisition unit 11 may have a configuration that acquires a target image obtained by capturing, with the camera, an image in which a specimen cell group to be classified as benign or malignant in the rapid on-site evaluation is projected with the microscope and the parameter indicating the effective magnification that is the magnification of the size of the subject in the target image, with respect to the actual subject size.

The determination unit 12 determines whether it is necessary to correct the parameter acquired by the acquisition unit 11. The determination unit 12 supplies the determination result to the notification unit 13 and the classification unit 14.

In a case where the determination unit 12 determines that the correction is necessary, the notification unit 13 notifies that it is necessary to correct the parameter.

In a case where the determination unit 12 determines that the correction is not necessary, the classification unit 14 performs benign/malignant classification on the specimen cell, using the parameter.

In a case where the acquisition unit 11 acquires the target image obtained by capturing, with the camera, the image in which the specimen cell group to be classified as benign or malignant in the rapid on-site evaluation is projected with the microscope, in a case where the determination unit 12 determines that the correction is not necessary, the classification unit 14 may perform benign/malignant classification on each specimen cell of the specimen cell group, using the parameter.

### (Effect of Pathological diagnosis support apparatus)

As described above, the pathological diagnosis support apparatus 1 adopts the configuration including the acquisition unit 11 that acquires the target image obtained by capturing, with the camera, the image in which the specimen cell to be classified as benign or malignant in the rapid on-site evaluation is projected with the microscope and the parameter indicating the effective magnification that is the magnification of the size of the subject in the target image, with respect to the actual subject size, the determination unit 12 that determines whether it is necessary to correct the parameter acquired by the acquisition unit 11, the notification unit 13 that notifies that it is necessary to correct the parameter, in a case where the determination unit 12 determines that the correction is necessary, and the classification unit 14 that performs benign/malignant classification on the specimen cell, using the parameter, in a case where the determination unit 12 determines that the correction is not necessary.

Therefore, according to the pathological diagnosis support apparatus 1, an effect is obtained that it is possible to provide the technology for improving accuracy in the benign/malignant classification of the specimen cell.

The pathological diagnosis support apparatus 1 may adopt the configuration including the acquisition unit 11 that acquires the target image obtained by capturing, with the camera, the image in which the specimen cell group to be classified as benign or malignant in the rapid on-site evaluation is projected with the microscope and the parameter indicating the effective magnification that is the magnification of the size of the subject in the target image, with respect to the actual subject size, the determination unit 12 that determines whether it is necessary to correct the parameter acquired by the acquisition unit 11, the notification unit 13 that notifies that it is necessary to correct the parameter, in a case where the determination unit 12 determines that the correction is necessary, and the classification unit 14 that performs benign/malignant classification on each specimen cell of the specimen cell group, using the parameter, in a case where the determination unit 12 determines that the correction is not necessary.

Therefore, according to the pathological diagnosis support apparatus 1, an effect is obtained that it is possible to provide the technology for improving accuracy in the benign/malignant classification for each specimen cell of the specimen cell group.

### (Flow of Pathological Diagnosis Support Method)

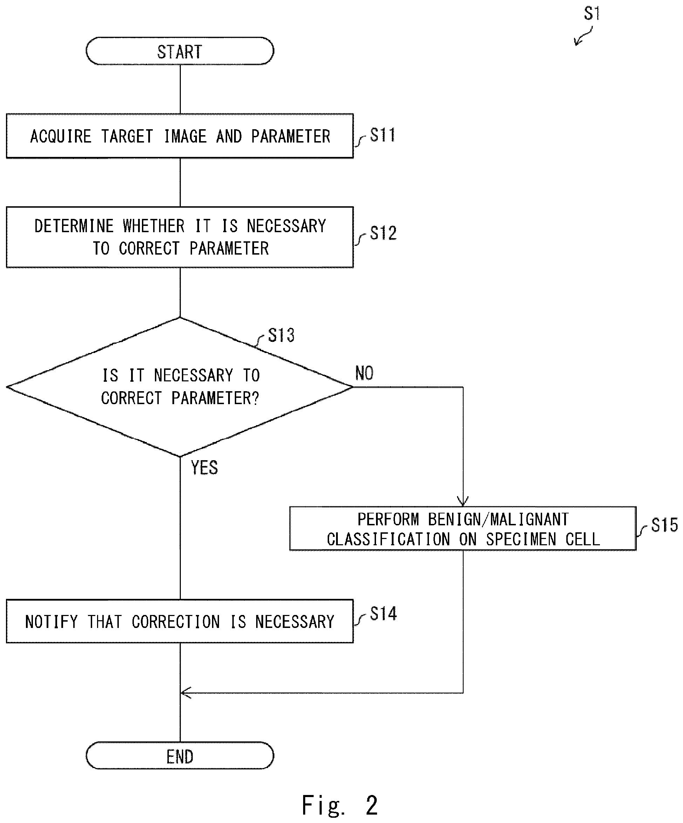

A flow of a pathological diagnosis support method S1 will be described with reference to Fig. 2. Fig. 2 is a flowchart illustrating the flow of the pathological diagnosis support method S1. As illustrated in Fig. 2, the pathological diagnosis support method S1 includes acquisition processing S11, determination processing S12, notification processing S14, and classification processing S15.

### (Acquisition Processing S11)

In the acquisition processing S11, the acquisition unit 11 acquires the target image obtained by capturing, with the camera, the image in which the specimen cell to be classified as benign or malignant in the rapid on-site evaluation is projected with the microscope and the parameter indicating the effective magnification that is the magnification of the size of the subject in the target image, with respect to the actual subject size. The acquisition unit 11 supplies the acquired target image to the classification unit 14 and supplies the acquired parameter to the determination unit 12.

In the acquisition processing S11, the acquisition unit 11 may have the configuration that acquires the target image obtained by capturing, with the camera, the image in which the specimen cell group to be classified as benign or malignant in the rapid on-site evaluation is projected with the microscope and the parameter indicating the effective magnification that is the magnification of the size of the subject in the target image, with respect to the actual subject size.

### (Determination Processing S12)

In the determination processing S12, the determination unit 12 determines whether it is necessary to correct the parameter acquired by the acquisition unit 11. The determination unit 12 supplies the determination result to the notification unit 13 and the classification unit 14.

### (Step S13)

In step S13, the notification unit 13 and the classification unit 14 determine whether the determination unit 12 determines that it is necessary to correct the parameter.

### (Notification Processing S14)

In a case where the determination unit 12 determines that the correction is necessary in the notification processing S13 (step S13: YES), the notification unit 13 notifies that it is necessary to correct the parameter in the notification processing S14.

### (Classification Processing S15)

In a case where the determination unit 12 determines in step S13 that the correction is not necessary (step S13: NO), in the classification processing S15, the classification unit 14 performs benign/malignant classification on the specimen cell, using the parameter.

In a case where the acquisition unit 11 acquires the target image obtained by capturing, with the camera, the image in which the specimen cell group to be classified as benign or malignant in the rapid on-site evaluation is projected with the microscope in the acquisition processing S11, in a case where the determination unit 12 determines that the correction is not necessary, the classification unit 14 may perform benign/malignant classification on each specimen cell of the specimen cell group, using the parameter in the classification processing S15.

### (Effect of Pathological Diagnosis Support Method)

As described above, the pathological diagnosis support method S1 adopts the configuration including the acquisition processing S11 by the acquisition unit 11 for acquiring the target image obtained by capturing, with the camera, the image in which the specimen cell to be classified as benign or malignant in the rapid on-site evaluation is projected with the microscope and the parameter indicating the effective magnification that is the magnification of the size of the subject in the target image, with respect to the size of the actual subject, the determination processing S12 by the determination unit 12 for determining whether it is necessary to correct the parameter acquired by the acquisition unit 11, the notification processing S14 by the notification unit 13 for notifying that it is necessary to correct the parameter, in a case where the determination unit 12 determines that the correction is necessary, and the classification processing S15 by the classification unit 14 for performing benign/malignant classification on the specimen cell, using the parameter, in a case where the determination unit 12 determines that the correction is not necessary.

Therefore, according to the pathological diagnosis support method S1, an effect similar to the pathological diagnosis support apparatus 1 described above is obtained.

The pathological diagnosis support method S1 may adopt the configuration including the acquisition processing S11 by the acquisition unit 11 for acquiring the target image obtained by capturing, with the camera, the image in which the specimen cell group to be classified as benign or malignant in the rapid on-site evaluation is projected with the microscope and the parameter indicating the effective magnification that is the magnification of the size of the subject in the target image, with respect to the actual subject size, the determination processing S12 by the determination unit 12 for determining whether it is necessary to correct the parameter acquired by the acquisition unit 11, the notification processing S14 by the notification unit 13 for notifying that it is necessary to correct the parameter, in a case where the determination unit 12 determines that the correction is necessary, and the classification processing S15 by the classification unit 14 for performing benign/malignant classification on each specimen cell of the specimen cell group, using the parameter, in a case where the determination unit 12 determines that the correction is not necessary.

Therefore, according to the pathological diagnosis support method S1, the effect is obtained that it is possible to provide the technology for improving the accuracy in the benign/malignant classification for each one specimen cell of the specimen cell group.

### [Second Exemplary Example Embodiment]

A second exemplary example embodiment that is an example of the example embodiments of the present invention will be described in detail with reference to the drawings. Components having the same functions as the components described in the above-described exemplary example embodiment will be denoted by the same reference signs, and description of the components will be appropriately omitted. An application range of each technical means adopted in the present exemplary example embodiment is not limited to the present exemplary example embodiment. That is, each technical means adopted in the present exemplary example embodiment can also be adopted in another exemplary example embodiment included in the present disclosure as long as no particular technical problem occurs. Each technical means illustrated in each of the drawings referred to for describing the present exemplary example embodiment can also be adopted in another exemplary example embodiment included in the present disclosure as long as no particular technical problem occurs.

### (Outline of Pathological diagnosis support apparatus 2)

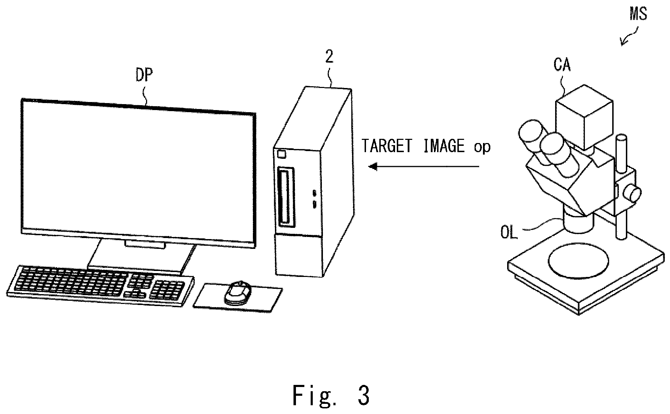

An outline of a pathological diagnosis support apparatus 2 will be described with reference to Fig. 3. Fig. 3 is a diagram illustrating an example of a state of pathological diagnosis using the pathological diagnosis support apparatus 2.

The pathological diagnosis support apparatus 2 is a device that supports rapid on-site evaluation. As an example, as illustrated in Fig. 3, the pathological diagnosis support apparatus 2 acquires a target image op including a specimen cell group as a subject, from a camera CA. The pathological diagnosis support apparatus 2 performs benign/malignant classification of whether each specimen cell of the specimen cell group included in the target image op as the subject is benign or malignant.

Similarly to the pathological diagnosis support apparatus 1 according to the exemplary example embodiment described above, the pathological diagnosis support apparatus 2 may have a configuration for acquiring the target image op including the specimen cell as the subject from the camera CA. With this configuration, the pathological diagnosis support apparatus 2 performs the benign/malignant classification of whether the specimen cell included in the target image op as the subject is benign or malignant. Hereinafter, the specimen cell group may be the single specimen cell, and the benign/malignant classification performed on each specimen cell of the specimen cell group may be benign/malignant classification performed on the single specimen cell.

As illustrated in Fig. 3, the camera CA is attached to a microscope MS. The camera CA captures an image obtained by enlarging the specimen cell group by the microscope MS using an objective lens OL. The camera CA outputs the target image op obtained by capturing the image, to the pathological diagnosis support apparatus 2. Although a magnification of the objective lens OL of the microscope MS is not particularly limited, the magnification is 40 times, as an example.

The pathological diagnosis support apparatus 2 standardizes a size of the subject included in the target image op and performs the benign/malignant classification on each specimen cell of the specimen cell group, using the standardized target image op. Here, the "standardization" refers to matching the size of the subject between a plurality of images. In order to standardize the size of the target image op, the pathological diagnosis support apparatus 2 applies a parameter indicating an effective magnification to the target image op. Here, the effective magnification means a magnification of the size of the subject in the target image op, with respect to an actual subject size. Although the parameter indicating the effective magnification is not particularly limited as long as the parameter indicates the effective magnification, the parameter may be an effective magnification correction parameter (actual measurement calibration value), in one aspect. The effective magnification correction parameter is a parameter indicating a length of the subject per pixel in the target image op and may be, for example, a parameter generated by imaging a prescribed micrometer.

In this configuration, in a case of determining whether it is necessary to correct the parameter and determines that the correction is necessary, the pathological diagnosis support apparatus 2 notifies that it is necessary to correct the parameter. As an example, as illustrated in Fig. 3, the pathological diagnosis support apparatus 2 makes a display indicating that it is necessary to correct the parameter, on a display DP. As another example, the pathological diagnosis support apparatus 2 may notify that it is necessary to correct the parameter using voice. On the other hand, in a case of determining that it is not necessary to correct the parameter, the pathological diagnosis support apparatus 2 applies the parameter to the target image op and performs the benign/malignant classification on each specimen cell of the specimen cell group.

### (Configuration of Pathological diagnosis support apparatus 2)

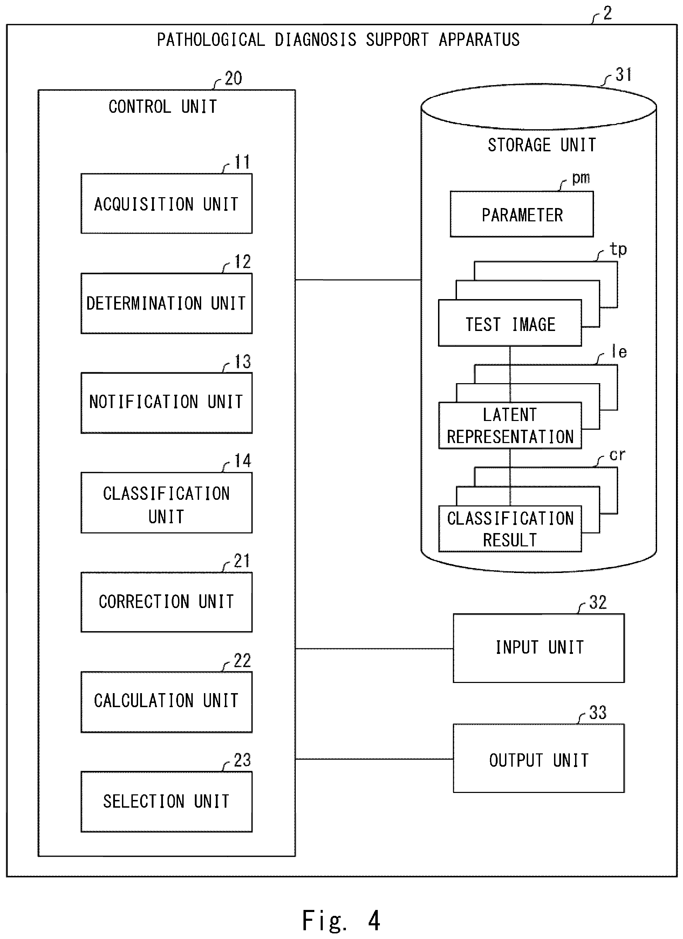

A configuration of the pathological diagnosis support apparatus 2 will be described with reference to Fig. 4. Fig. 4 is a block diagram illustrating the configuration of the pathological diagnosis support apparatus 2. As illustrated in Fig. 4, the pathological diagnosis support apparatus 2 includes a control unit 20, a storage unit 31, an input unit 32, and an output unit 33.

The storage unit 31 stores data referred to by the control unit 20. As an example, the storage unit 31 stores a parameter pm, a test image tp, a latent representation le, and a classification result cr to be described later in association with each other. Examples of the storage unit 31 include, but are not limited to, a flash memory, a Hard Disk Drive (HDD), a Solid State Drive (SSD), and a combination thereof.

The input unit 32 receives an input of data. The input unit 32 supplies the received data to the control unit 20. Examples of the data received by the input unit 32 include the target image op imaged by the camera CA.

The output unit 33 outputs the data supplied from the control unit 20. As an example, the output unit 33 outputs an image to the display DP.

### (Control Unit 20)

The control unit 20 controls each component included in the pathological diagnosis support apparatus 2. As illustrated in Fig. 4, the control unit 20 includes an acquisition unit 11, a determination unit 12, a notification unit 13, a classification unit 14, a correction unit 21, a calculation unit 22, and a selection unit 23. That is, the pathological diagnosis support apparatus 2 includes the correction unit 21, the calculation unit 22, and the selection unit 23, in addition to the acquisition unit 11, the determination unit 12, the notification unit 13, and the classification unit 14 included in the pathological diagnosis support apparatus 1. The acquisition unit 11, the determination unit 12, the notification unit 13, the classification unit 14, the correction unit 21, the calculation unit 22, and the selection unit 23 are configurations respectively achieving acquisition means, determination means, notification means, classification means, correction means, calculation means, and selection means, in the present exemplary example embodiment.

The acquisition unit 11 acquires data. The acquisition unit 11 stores the acquired data in the storage unit 31.

As an example, the acquisition unit 11 acquires the target image op obtained by capturing, with the camera CA, an image in which a specimen cell group to be classified as benign or malignant in the rapid on-site evaluation is projected with the microscope MS and a parameter pm indicating the effective \magnification that is the magnification of the size of the subject in the target image op, with respect to the actual subject size. As another example, the acquisition unit 11 acquires a generation date and time at which the parameter pm is generated. As still another example, the acquisition unit 11 acquires a shipping date and time of the camera CA. As yet another example, the acquisition unit 11 acquires an attachment/detachment date and time at which the camera CA is attached/detached to/from the microscope MS.

As an example of a method for acquiring the data by the acquisition unit 11, a method for acquiring the data from a user via the input unit 32 is exemplified. As another example of the method for acquiring the data by the acquisition unit 11, a method for acquiring the data from the camera CA via the input unit 32 is exemplified.

The determination unit 12 determines whether it is necessary to correct the parameter pm. The determination unit 12 stores the determination result in the storage unit 31. An example of processing executed by the determination unit 12 will be described later.

In a case where the determination unit 12 determines that the correction is necessary, the notification unit 13 notifies that it is necessary to correct the parameter pm. As an example, the notification unit 13 makes a display indicating that it is necessary to correct the parameter pm, on the display DP via the output unit 33.

The classification unit 14 performs the benign/malignant classification on the specimen cell group. As an example, in a case where the determination unit 12 determines that the correction is not necessary, the size of the target image op is standardized using the parameter pm, and the benign/malignant classification is performed on each specimen cell of the specimen cell group included in the standardized target image op as the subject.

For example, in a case where the standardized size indicates that a length per pixel is x [nm] and a length per pixel of the parameter pm of the target image op is y [nm], the classification unit 14 performs standardization by multiplying the target image op by y/x.

As an example of a method for performing the benign/malignant classification by the classification unit 14, a configuration using a machine learned classification model is exemplified. In this case, by inputting the target image op to which the parameter pm is applied into the classification model machine learned to detect each cell of the specimen cell group and classify whether each cell is benign or malignant, using an image including the specimen cell group as the subject as an input, the classification unit 14 performs the benign/malignant classification on each specimen cell of the specimen cell group included in the target image op as the subject. With this configuration, since the classification unit 14 can input the standardized target image op into the classification model machine learned using the standardized image, it is possible to suitably perform the benign/malignant classification.

As described above, the classification unit 14 may have a configuration for performing the benign/malignant classification on the specimen cell included in the target image op as the subject, by inputting the target image op to which the parameter pm is applied, into the classification model machine learned to classify whether the specimen cell is benign or malignant, using the image including the specimen cell as the subject as an input.

The correction unit 21 corrects the parameter pm. The correction unit 21 stores the corrected parameter pm in the storage unit 31. An example of processing executed by the correction unit 21 will be described later.

The calculation unit 22 calculates a similarity between each of a plurality of test images tp on which benign/malignant classification is performed by the classification unit 14 and the target image op. The calculation unit 22 stores the calculated similarity in the storage unit 31. The test image tp indicates an image in which each specimen cell of the specimen cell group included in the test image tp as the subject is classified to be benign or malignant in advance. The test image tp and the classification result cr may be stored in the storage unit 31 in association with each other, as illustrated in Fig. 4. An example of processing executed by the calculation unit 22 will be described later.

The selection unit 23 selects a predetermined number of test images tp of which the similarity calculated by the calculation unit 22 is high. The selection unit 23 stores the selection result in the storage unit 31. Although the predetermined number is not particularly limited, in a case where the number of test images tp is N, the predetermined number is N/10 as an example.

### (Pathological Diagnosis Support Method S2)

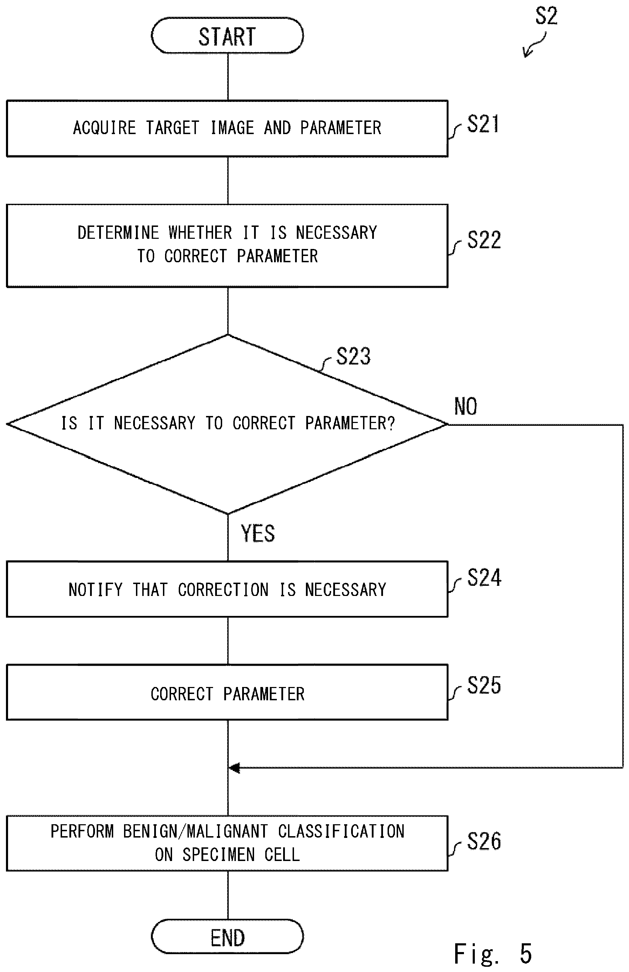

A flow of a pathological diagnosis support method S2 will be described with reference to Fig. 5. Fig. 5 is a flowchart illustrating the flow of the pathological diagnosis support method S2.

### (Step S21)

In step S21, the acquisition unit 11 acquires the target image op obtained by capturing, with the camera CA, the image in which the specimen cell group to be classified as benign or malignant in the rapid on-site evaluation is projected with the microscope MS and the parameter pm indicating the effective magnification that is the magnification of the size of the subject in the target image op, with respect to the actual subject size. The acquisition unit 11 stores the acquired target image op and parameter pm, in the storage unit 31.

### (Step S22)

In step S22, the determination unit 12 determines whether it is necessary to correct the parameter pm acquired by the acquisition unit 11. The determination unit 12 stores the determination result in the storage unit 31.

### (Step S23)

In step S23, the notification unit 13 and the classification unit 14 determine whether the determination unit 12 determines that it is necessary to correct the parameter pm, based on the determination result stored in the storage unit 31.

### (Step S24)

In a case where the determination unit 12 determines in step S23 that the correction is necessary (step S23: YES), the notification unit 13 notifies in step S24 that it is necessary to correct the parameter pm.

### (Step S25)

In step S25, the correction unit 21 corrects the parameter pm. That is, in a case where the determination unit 12 determines that the correction is necessary, the correction unit 21 corrects the parameter pm. The correction unit 21 stores the corrected parameter pm in the storage unit 31.

### (Step S26)

After step S25 is executed or in a case where the determination unit 12 determines in step S23 that the correction is not necessary (step S23: NO), the classification unit 14 performs benign/malignant classification, in step S26, on each specimen cell of the specimen cell group included in the target image op as the subject, using the parameter pm stored in the storage unit 31.

That is, in a case where the classification unit 14 executes step S26 after executing step S25, the classification unit 14 performs the benign/malignant classification on each specimen cell of the specimen cell group included in the target image op as the subject, using the parameter pm corrected by the correction unit 21. With this configuration, since the classification unit 14 performs the benign/malignant classification on each specimen cell of the specimen cell group using the corrected parameter pm in a case where it is determined that it is necessary to correct the parameter pm, it is possible to improve accuracy in the benign/malignant classification.

### (Example 1 of Processing Executed by Determination Unit 12)

An example of the processing executed by the determination unit 12 in step S22 will be described.

In a case where the acquisition unit 11 acquires the generation date and time at which the parameter pm stored in the storage unit 31 is generated, the determination unit 12 determines whether it is necessary to correct the parameter pm, based on the generation date and time.

As an example, in a case where the acquisition unit 11 acquires the shipping date and time of the camera CA and the generation date and time at which the parameter pm is generated is the shipping date and time of the camera CA, the determination unit 12 determines that it is necessary to correct the parameter pm. In this case, as an example, the notification unit 13 notifies a message "Please image a micrometer and perform effective magnification correction".

In this way, in a case where the correction unit 21 performs correction using the image of the micrometer, the notification unit 13 may issue a notification, including an indication that the micrometer is to be installed at a viewing angle of the microscope MS (that micrometer is to be imaged), in an indication that it is necessary to correct the parameter pm, in step S24 described above. With this configuration, the pathological diagnosis support apparatus 2 can suitably correct the parameter pm.

With this configuration, the pathological diagnosis support apparatus 2 can correct the parameter pm to a parameter pm according to an environment in which the camera CA is installed in the microscope MS.

As another example, in a case where the acquisition unit 11 acquires the attachment/detachment date and time of the camera CA and the generation date and time at which the parameter pm is generated is earlier than the attachment/detachment date and time of the camera CA, the determination unit 12 determines that it is necessary to correct the parameter pm. In this case, as an example, the notification unit 13 notifies a message "Effective magnification correction is not performed after attachment/detachment of the camera. Please image the micrometer again and perform effective magnification correction".

With this configuration, even if the effective magnification changes before and after the attachment/detachment of the camera CA, the pathological diagnosis support apparatus 2 can standardize the target image op.

As still another example, in a case where the generation date and time of the parameter pm is earlier than a predetermined date and time, the determination unit 12 determines that it is necessary to correct the parameter pm. In other words, in a case where the parameter pm is not updated for a predetermined period, the determination unit 12 determines that it is necessary to correct the parameter pm. Although the predetermined period is not limited, the predetermined period is 30 days, as an example. In this case, as an example, the notification unit 13 notifies a message "The parameter for effective magnification correction is not updated for a certain period. Latest update date and time is t:u on Y/Z/XXXX. If a microscope camera is attached/detached on and after the latest update date and time, please image the micrometer again and perform effective magnification correction".

With this configuration, even if the effective magnification changes with time, the pathological diagnosis support apparatus 2 can standardize the target image op.

In this way, the determination unit 12 determines whether it is necessary to correct the parameter pm, based on the generation date and time. Therefore, in a case where the effective magnification is changed by installing the camera CA, in a case where the effective magnification is changed by attaching/detaching the camera CA, or in a case where the effective magnification is changed with time, the determination unit 12 can correct the parameter pm.

### (Example 2 of Processing Executed by Determination Unit 12)

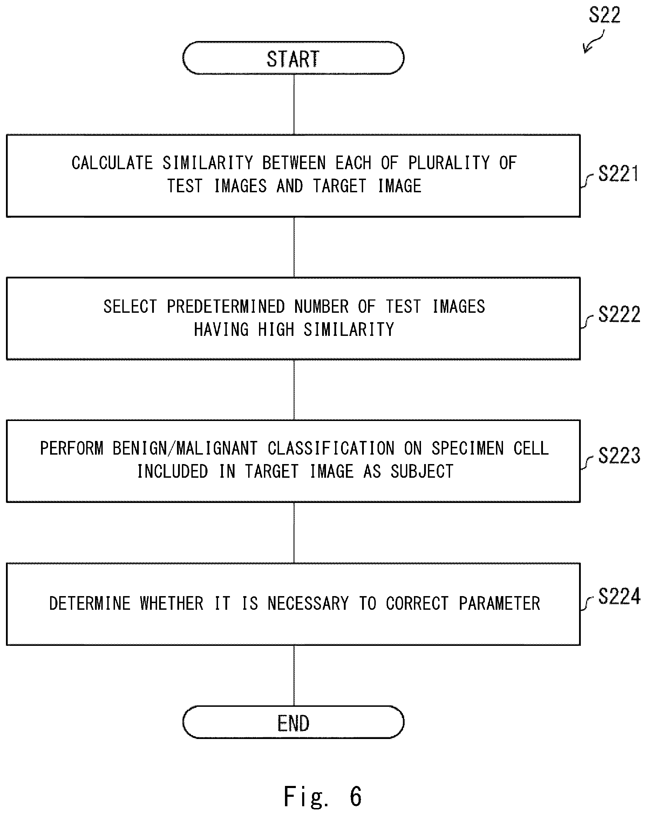

Another example of the processing executed by the determination unit 12 in step S22 will be described with reference to Fig. 6. Fig. 6 is a flowchart illustrating a flow of the processing executed by the determination unit 12.

### (Step S221)

In step S221, the calculation unit 22 calculates the similarity between each of the plurality of test images tp stored in the storage unit 31 and the target image op.

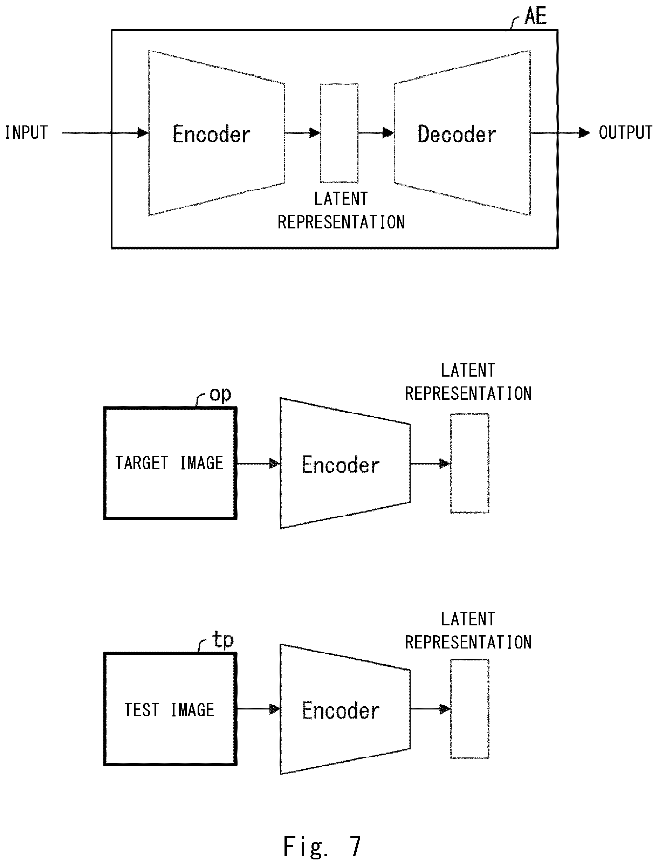

An example of the processing for calculating the similarity by the calculation unit 22 in step S221 will be described with reference to Fig. 7. Fig. 7 is a diagram illustrating an example of the processing for calculating the similarity by the calculation unit 22.

As an example, the calculation unit 22 calculates the similarity, using an autoencoder AE. As illustrated in an upper portion of Fig. 7, the autoencoder AE includes an Encoder that is machine learned to compress a dimension of an image using the image as an input and outputs a latent representation that is an important feature representation and a Decoder that is machine learned to output an image obtained by decoding to the original dimension, using the latent representation as an input. As an example, the latent representation is represented by a vector (for example, 64 vectors).

The calculation unit 22 inputs the target image op to the autoencoder and acquires a latent representation le of the target image op. Similarly, the calculation unit 22 inputs the test image tp to the autoencoder and acquires the latent representation le of the test image tp.

Before step S221 is executed, the calculation unit 22 may acquire the latent representation le of each of the plurality of test images tp. In this case, as illustrated in Fig. 4, the calculation unit 22 may store each test image tp and the latent representation le in the storage unit 31, in association with each other.

Then, the calculation unit 22 calculates the similarity, based on a difference between the latent representation le of the target image op and the latent representation le of the test image tp. As an example, the calculation unit 22 calculates a Cosine similarity as the similarity between the target image op and each test image tp, using the following formula (1).
[Mathematical Formula 1] $Cosine similarity \left(\vec{A}, \vec{B}\right) = \frac{\vec{A} ⋅ \vec{B}}{\left‖\vec{A}\right‖ \left‖\vec{B}\right‖} = \frac{{\sum }_{n} {A}_{n} {B}_{n}}{\sqrt{{\sum }_{n} {A}_{n}^{2}} \sqrt{{\sum }_{n} {B}_{n}^{2}}}$
A̅: LATENT REPRESENTATION OF TARGET IMAGE op
B̅: LATENT REPRESENTATION OF TEST IMAGE tp

With this configuration, the calculation unit 22 can suitably calculate the similarity between the target image op and the test image tp.

### (Step S222)

In step S222, the selection unit 23 selects the predetermined number of test images tp of which the similarity calculated by the calculation unit 22 is high.

### (Step S223)

In step S223, the classification unit 14 performs the benign/malignant classification on each specimen cell of the specimen cell group included in the target image op as the subject.

### (Step S224)

In step S224, the determination unit 12 determines whether it is necessary to correct the parameter pm, based on a difference between the classification result cr of each of the predetermined number of test images tp selected in step S222 and the classification result cr of the specimen cell group included in the target image op as the subject in step S223.

As an example, in a case where the classification result cr is a result of classification by the classification unit 14 using the classification model and a probability that each specimen cell of the specimen cell group is malicious, in a case where a mean squared error between a probability of the classification result cr of the predetermined number of test images tp and a probability of the classification result cr of the target image op is equal to or more than a threshold, the determination unit 12 determines that it is necessary to correct the parameter pm.

In other words, in a case where the difference between the classification result cr of the test image tp similar to the target image op and the classification result cr of the target image op is large, the determination unit 12 determines that it is necessary to correct the parameter pm.

In this way, the pathological diagnosis support apparatus 2 determines whether it is necessary to correct the parameter pm, based on the difference between the classification result cr of the test image tp similar to the target image op and the classification result cr of the target image op. Specifically, in a case where the difference between the classification result cr of the test image tp similar to the target image op and the classification result cr of the target image op is large, the classification results cr are greatly different, although the target image op and the test image tp are similar to each other. In such a case, there is a possibility that effective magnifications at which the target image op and the test image tp are imaged are different. In such a case, it can be determined that it is necessary to correct the parameter pm.

The determination unit 12 may have a configuration in which the configuration in this example and the configuration for making determination based on the generation date and time at which the parameter pm is generated are combined. For example, the determination unit 12 may have a configuration for executing the processing illustrated in Fig. 6, in a case of determining that the determination result based on the generation date and time indicates that it is not necessary to correct the parameter pm.

### (Example of Processing Executed by Correction Unit 21)

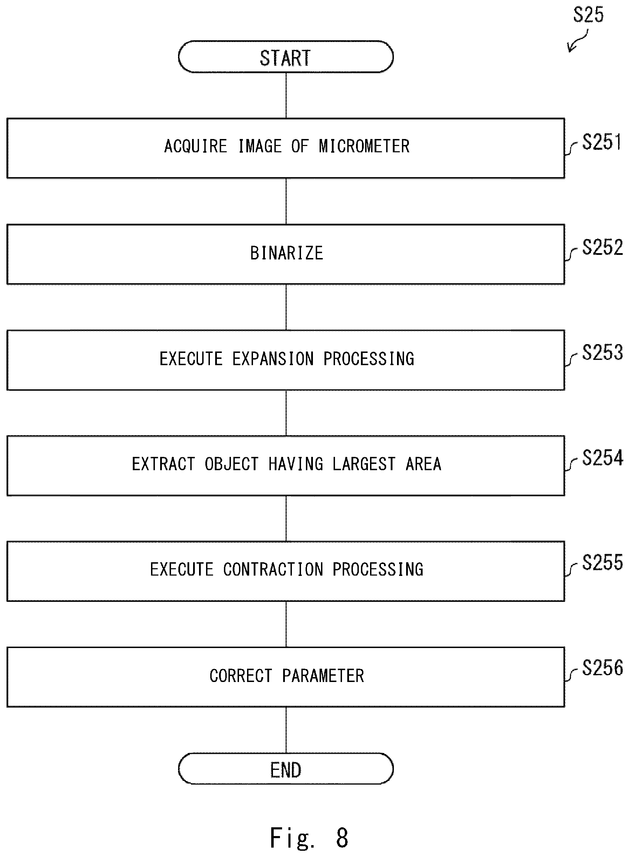

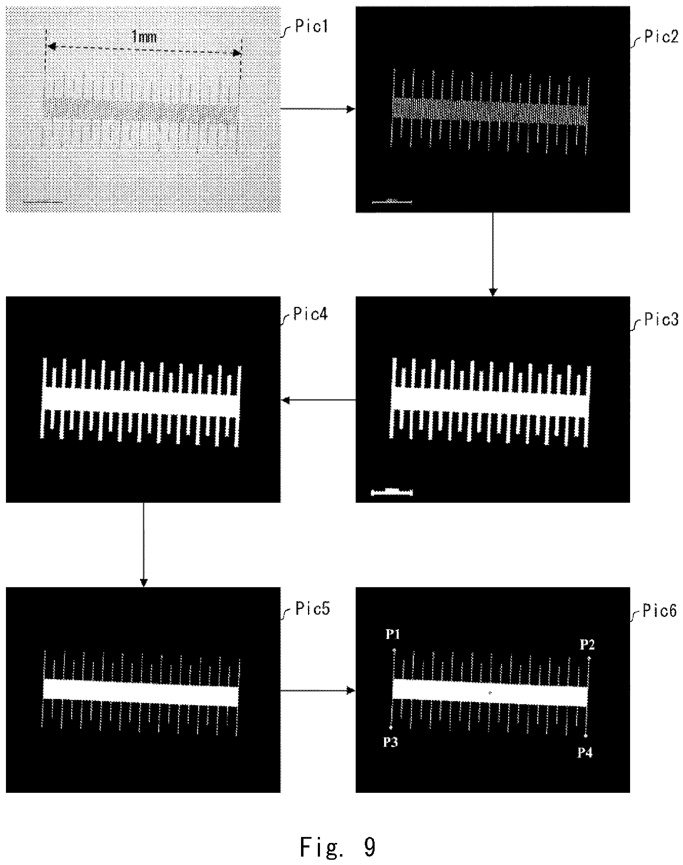

An example of the processing executed by the correction unit 21 in step S25 will be described with reference to Figs. 8 and 9. Fig. 8 is a flowchart illustrating a flow of the processing executed by the correction unit 21. Fig. 9 is an example of an image processed by the correction unit 21.

### (Step S251)

In step S251, the acquisition unit 11 acquires an image including the micrometer as the subject. The correction unit 21 acquires the image of the micrometer acquired by the acquisition unit 11. For example, as illustrated in Fig. 9, the correction unit 21 acquires an image Pic1 including a micrometer having a length of 1 mm as the subject.

### (Step S252)

In step S252, the correction unit 21 binarizes the image of the micrometer. For example, as illustrated in Fig. 9, the correction unit 21 generates an image Pic2 obtained by binarizing the image of the micrometer.

### (Step S253)

In step S253, the correction unit 21 executes expansion processing on the image generated in step S252. For example, as illustrated in Fig. 9, the correction unit 21 generates an image Pic3 obtained by executing the expansion processing on the binarized image Pic2.

### (Step S254)

In step S254, the correction unit 21 extracts an object having the largest area, in the image on which the expansion processing has been executed in step S253. For example, as illustrated in Fig. 9, the correction unit 21 generates an image Pic4 in which the micrometer is maximized, in the image Pic3 on which the expansion processing has been executed.

### (Step S255)

In step S255, the correction unit 21 executes contraction processing, on the image generated in step S254. For example, as illustrated in Fig. 9, the correction unit 21 generates an image Pic5 obtained by executing the contraction processing on the image Pic4 in which the micrometer is maximized.

### (Step S256)

In step S256, the correction unit 21 corrects the parameter pm.

For example, as illustrated in Fig. 9, the correction unit 21 detects a point P1, a point P2, a point P3, and a point P4 that are four ends of the micrometer, in the image Pic5 on which the contraction processing has been executed. In other words, the point P1, the point P2, the point P3, and the point P4 that are points farthest from the center of the micrometer are detected. Subsequently, the correction unit 21 calculates the corrected parameter (length [nm] per pixel), using the following formula (2). $Corrected parameter = 1.0 \times {10}^{6} / Ave \left(d_P12+d_P34\right)$
d_P12: the number of pixels from the point P1 to the point P2.
d_P34: the number of pixels from the point P1 to the point P2.

### (Effect of Pathological diagnosis support apparatus 2)

In this way, in a case where it is necessary to correct the parameter pm to be applied to the target image op, the pathological diagnosis support apparatus 2 corrects the parameter pm, after notifying that it is necessary to correct the parameter pm. Then, the pathological diagnosis support apparatus 2 performs pathological diagnosis on each specimen cell of the specimen cell group, using the corrected parameter pm.

For example, in a case where the plurality of target images op is imaged on different dates and times, in a case where the plurality of target images op is imaged using the different microscopes MS, and in a case where the plurality of target images op is imaged using the different cameras CA, there is a possibility that the effective magnifications of the target images op are different. In such a case, the pathological diagnosis support apparatus 2 can standardize the plurality of target images op and make the subject having the same size have the same size between the plurality of target images op.

Therefore, the pathological diagnosis support apparatus 2 can improve the accuracy in the benign/malignant classification on each specimen cell of the specimen cell group, in the pathological diagnosis.

In particular, in a case where the classification model is used that is machine learned to detect each cell of the specimen cell group and classify whether each cell is benign or malignant, if the effective magnification of the image used for training of the classification model and the effective magnification of the target image op are different, the accuracy in the benign/malignant classification by the classification model decreases. As described above, the pathological diagnosis support apparatus 2 can standardize the image used to train the classification model and the target image op. Therefore, the pathological diagnosis support apparatus 2 can improve the accuracy in the benign/malignant classification on each specimen cell of the specimen cell group, even in a case of using the classification model.

### [Implementation Example by Software]

Some or all of the functions of the pathological diagnosis support apparatuses 1 and 2 (hereinafter also referred to as "the above apparatuses") may be achieved by hardware such as an Integrated Circuit (IC chip), or may be achieved by software.

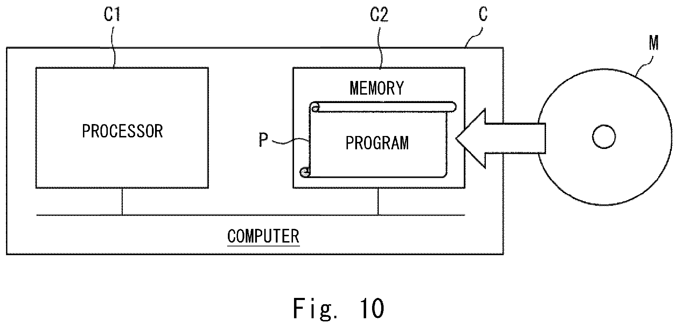

In the latter case, each of the above apparatuses is achieved by, for example, a computer that executes commands of a program that is software for achieving each function. An example of such a computer (hereinafter, referred to as computer C) is illustrated in Fig. 10. Fig. 10 is a block diagram illustrating a hardware configuration of the computer C that functions as each of the above apparatuses.

The computer C includes at least one processor C1 and at least one memory C2. A program P for causing the computer C to operate as each of the above apparatuses is recorded in the memory C2. In the computer C, by the processor C1 reading the program P from the memory C2 and executing the program P, each of the functions of each of the above apparatuses is achieved.

As the processor C1, for example, a Central Processing Unit (CPU), a Graphic Processing Unit (GPU), a Digital Signal Processor (DSP), a Micro Processing Unit (MPU), a Floating point number Processing Unit (FPU), a Physics Processing Unit (PPU), a Tensor Processing Unit (TPU), a quantum processor, a microcontroller, or a combination of these can be used. As the memory C2, for example, a flash memory, a Hard Disk Drive (HDD), a Solid State Drive (SSD), or a combination of these can be used.

The computer C may further include a Random Access Memory (RAM) for loading the program P at the time of execution and temporarily storing various types of data. The computer C may further include a communication interface for transmitting and receiving data to and from another device. The computer C may further include an input/output interface for connecting input/output devices such as a keyboard, a mouse, a display, or a printer.

The program P can be recorded in a non-transitory tangible recording medium M readable by the computer C. As such a recording medium M, for example, a tape, a disk, a card, a semiconductor memory, a programmable logic circuit, or the like can be used. The computer C can acquire the program P via such a recording medium M. The program P can be transmitted via a transmission medium. As such a transmission medium, for example, a communication network, a broadcast wave, or the like can be used. The computer C can also acquire the program P via such a transmission medium.

### [Supplementary Information A]

The present disclosure includes the technologies described in the following Supplementary Notes. However, the present invention is not limited to the technologies described in the following Supplementary Notes, and various modifications can be made within the scope described in the claims.

### (Supplementary Note A1)

A pathological diagnosis support apparatus including:
acquisition means for acquiring a target image obtained by capturing, with a camera, an image in which a specimen cell to be classified as benign or malignant in rapid on-site evaluation is projected with a microscope and a parameter indicating an effective magnification that is a magnification of a size of a subject in the target image, with respect to an actual subject size;
determination means for determining whether it is necessary to correct the parameter;
notification means for notifying that it is necessary to correct the parameter, in a case where the determination means determines that the correction is necessary; and
classification means for performing benign/malignant classification on the specimen cell, using the parameter, in a case where the determination means determines that the correction is not necessary.

### (Supplementary Note A2)

The pathological diagnosis support apparatus according to supplementary note A1, further including correction means for correcting the parameter, in which
in a case where the determination means determines that the correction is necessary,
the correction means corrects the parameter, and
the classification means performs benign/malignant classification on the specimen cell, using the parameter corrected by the correction means.

### (Supplementary Note A3)

The pathological diagnosis support apparatus according to supplementary note A1 or A2, in which the notification means includes an indication that a micrometer is to be installed at a viewing angle in the microscope, in an indication that it is necessary to correct the parameter.

### (Supplementary Note A4)

The pathological diagnosis support apparatus according to any one of supplementary notes A1 to A3, in which the classification means performs benign/malignant classification on the specimen cell included in the target image as the subject, by inputting the target image into a classification model machine learned to classify whether the specimen cell is benign or malignant, using an image including the specimen cell as the subject, as an input.

### (Supplementary Note A5)

The pathological diagnosis support apparatus according to any one of supplementary notes A1 to A4, in which
the acquisition means further acquires a generation date and time at which the parameter is generated, and
the determination means determines whether it is necessary to correct the parameter, based on the generation date and time.

### (Supplementary Note A6)

The pathological diagnosis support apparatus according to supplementary note A5, in which
the acquisition means further acquires a shipping date and time of the camera, and
in a case where the generation date and time is the shipping date and time, the determination means determines that it is necessary to correct the parameter.

### (Supplementary Note A7)

The pathological diagnosis support apparatus according to supplementary note A5, in which
the acquisition means further acquires an attachment/detachment date and time at which the camera is attached/detached, and
in a case where the generation date and time is earlier than the attachment/detachment date and time, the determination means determines that it is necessary to correct the parameter.

### (Supplementary Note A8)

The pathological diagnosis support apparatus according to supplementary note A5, in which, in a case where the generation date and time is earlier than a predetermined date and time, the determination means determines that it is necessary to correct the parameter.

### (Supplementary Note A9)

The pathological diagnosis support apparatus according to any one of supplementary notes A1 to A4, further including:
calculation means for calculating a similarity between each of a plurality of test images on which benign/malignant classification is performed by the classification means and the target image; and
selection means for selecting a predetermined number of test images having the high similarity, in which
the classification means performs benign/malignant classification on the specimen cell included in the target image as the subject, and
the determination means determines whether it is necessary to correct the parameter, based on a difference between a classification result of each of the plurality of test images selected by the selection means and a classification result of the specimen cell included in the target image as the subject.

### (Supplementary Note A10)

The pathological diagnosis support apparatus according to supplementary note A9, in which the calculation means calculates the similarity, based on a difference between a latent representation obtained by inputting each of the test images to an encoder that is machine learned to output a latent representation of an image, using the image as an input and a latent representation obtained by inputting the target image to the encoder.

### (Supplementary Note A11)

A pathological diagnosis support apparatus including:
acquisition means for acquiring a target image obtained by capturing, with a camera, an image in which a specimen cell group to be classified as benign or malignant in rapid on-site evaluation is projected with a microscope and a parameter indicating an effective magnification that is a magnification of a size of a subject in the target image, with respect to an actual subject size;
determination means for determining whether it is necessary to correct the parameter;
notification means for notifying that it is necessary to correct the parameter, in a case where the determination means determines that the correction is necessary; and
classification means for performing benign/malignant classification on each specimen cell of the specimen cell group, using the parameter, in a case where the determination means determines that the correction is not necessary.

### [Supplementary Information B]

The present disclosure includes the technologies described in the following Supplementary Notes. However, the present invention is not limited to the technologies described in the following Supplementary Notes, and various modifications can be made within the scope described in the claims.

### (Supplementary Note B1)

A pathological diagnosis support method performed by at least one processor, including:
acquisition processing for acquiring a target image obtained by capturing, with a camera, an image in which a specimen cell to be classified as benign or malignant in rapid on-site evaluation is projected with a microscope and a parameter indicating an effective magnification that is a magnification of a size of a subject in the target image, with respect to an actual subject size;
determination processing for determining whether it is necessary to correct the parameter;
notification processing for notifying that it is necessary to correct the parameter, in a case where it is determined in the determination processing that the correction is necessary; and
classification processing for performing benign/malignant classification on the specimen cell, using the parameter, in a case where it is determined in the determination processing that the correction is not necessary.

### (Supplementary Note B2)

The pathological diagnosis support method according to supplementary note B1, performed by the at least one processor, further including correction processing for correcting the parameter, in which
in a case where it is determined in the determination processing that the correction is necessary,
the at least one processor
corrects the parameter in the correction processing, and
performs benign/malignant classification on the specimen cell, using the parameter corrected in the correction processing, in the classification processing.

### (Supplementary Note B3)

The pathological diagnosis support method according to supplementary note B1 or B2, in which, in the notification processing, the at least one processor includes an indication that a micrometer is to be installed at a viewing angle in the microscope, in an indication that it is necessary to correct the parameter.

### (Supplementary Note B4)

The pathological diagnosis support method according to any one of supplementary notes B1 to B3, in which, in the classification processing, the at least one processor performs benign/malignant classification on the specimen cell included in the target image as the subject, by inputting the target image into a classification model machine learned to classify whether the specimen cell is benign or malignant, using an image including the specimen cell as the subject, as an input.

### (Supplementary Note B5)

The pathological diagnosis support method according to any one of supplementary notes B1 to B4, in which
the at least one processor
further acquires a generation date and time at which the parameter is generated in the acquisition processing, and
determines whether it is necessary to correct the parameter, based on the generation date and time in the determination processing.

### (Supplementary Note B6)

The pathological diagnosis support method according to supplementary note B5, in which
the at least one processor
further acquires a shipping date and time of the camera in the acquisition processing, and
in a case where the generation date and time is the shipping date and time, the at least one processor determines that it is necessary to correct the parameter, in the determination processing.

### (Supplementary Note B7)

The pathological diagnosis support method according to supplementary note B5, in which
the acquisition processing further acquires an attachment/detachment date and time at which the camera is attached/detached, and
in a case where the generation date and time is earlier than the attachment/detachment date and time, it is determined that it is necessary to correct the parameter, in the determination processing.

### (Supplementary Note B8)

The pathological diagnosis support method according to supplementary note B5, in which, in a case where the generation date and time is earlier than a predetermined date and time, the at least one processor determines that it is necessary to correct the parameter, in the determination processing.

### (Supplementary Note B9)

The pathological diagnosis support method according to any one of supplementary notes B1 to B4, performed by the at least one processor, further including:
calculation processing for calculating a similarity between each of a plurality of test images on which benign/malignant classification is performed in the classification processing and the target image; and
selection processing for selecting a predetermined number of test images having the high similarity, in which
the at least one processor performs benign/malignant classification on the specimen cell included in the target image as the subject, in the classification processing, and
the at least one processor determines, in the determination processing, whether it is necessary to correct the parameter, based on a difference between a classification result of each of the plurality of test images selected in the selection processing and a classification result of the specimen cell included in the target image as the subject.

### (Supplementary Note B10)

The pathological diagnosis support method according to supplementary note B9, in which, in the calculation processing, the at least one processor calculates the similarity, based on a difference between a latent representation obtained by inputting each of the test images to an encoder that is machine learned to output a latent representation of an image, using the image as an input and a latent representation obtained by inputting the target image to the encoder.

### (Supplementary Note B11)

A pathological diagnosis support method performed by at least one processor, including:
acquisition processing for acquiring a target image obtained by capturing, with a camera, an image in which a specimen cell group to be classified as benign or malignant in rapid on-site evaluation is projected with a microscope and a parameter indicating an effective magnification that is a magnification of a size of a subject in the target image, with respect to an actual subject size;
determination processing for determining whether it is necessary to correct the parameter;
notification processing for notifying that it is necessary to correct the parameter, in a case where it is determined in the determination processing that the correction is necessary; and
classification processing for performing benign/malignant classification on each specimen cell of the specimen cell group, using the parameter, in a case where it is determined in the determination processing that the correction is not necessary.

### [Supplementary Information C]

The present disclosure includes the technologies described in the following Supplementary Notes. However, the present invention is not limited to the technologies described in the following Supplementary Notes, and various modifications can be made within the scope described in the claims.

### (Supplementary Note C1)

A pathological diagnosis support program for causing a computer to function as a pathological diagnosis support apparatus, the program for causing the computer to function as:
acquisition means for acquiring a target image obtained by capturing, with a camera, an image in which a specimen cell to be classified as benign or malignant in rapid on-site evaluation is projected with a microscope and a parameter indicating an effective magnification that is a magnification of a size of a subject in the target image, with respect to an actual subject size;
determination means for determining whether it is necessary to correct the parameter;
notification means for notifying that it is necessary to correct the parameter, in a case where the determination means determines that the correction is necessary; and
classification means for performing benign/malignant classification on the specimen cell, using the parameter, in a case where the determination means determines that the correction is not necessary.

### (Supplementary Note C2)

The pathological diagnosis support program according to supplementary note C1, for causing the computer to further function as correction means for correcting the parameter, in which
in a case where the determination means determines that the correction is necessary,
the correction means corrects the parameter, and
the classification means performs benign/malignant classification on the specimen cell, using the parameter corrected by the correction means.

### (Supplementary Note C3)

The pathological diagnosis support program according to supplementary note C1 or C2, in which the notification means includes an indication that a micrometer is to be installed at a viewing angle in the microscope, in an indication that it is necessary to correct the parameter.

### (Supplementary Note C4)

The pathological diagnosis support program according to any one of supplementary notes C1 to C3, in which the classification means performs benign/malignant classification on the specimen cell included in the target image as the subject, by inputting the target image into a classification model machine learned to classify whether the specimen cell is benign or malignant, using an image including the specimen cell as the subject, as an input.

### (Supplementary Note C5)

The pathological diagnosis support program according to any one of supplementary notes C1 to C4, in which
the acquisition means further acquires a generation date and time at which the parameter is generated, and
the determination means determines whether it is necessary to correct the parameter, based on the generation date and time.

### (Supplementary Note C6)

The pathological diagnosis support program according to supplementary note C5, in which
the acquisition means further acquires a shipping date and time of the camera, and
in a case where the generation date and time is the shipping date and time, the determination means determines that it is necessary to correct the parameter.

### (Supplementary Note C7)

The pathological diagnosis support program according to supplementary note C5, in which
the acquisition means further acquires an attachment/detachment date and time at which the camera is attached/detached, and
in a case where the generation date and time is earlier than the attachment/detachment date and time, the determination means determines that it is necessary to correct the parameter.

### (Supplementary Note C8)

The pathological diagnosis support program according to supplementary note C5, in which, in a case where the generation date and time is earlier than a predetermined date and time, the determination means determines that it is necessary to correct the parameter.

### (Supplementary Note C9)

The pathological diagnosis support program according to any one of supplementary notes C1 to C4, for causing the computer to further function as:
calculation means for calculating a similarity between each of a plurality of test images on which benign/malignant classification is performed by the classification means and the target image; and
selection means for selecting a predetermined number of test images having the high similarity, in which
the classification means performs benign/malignant classification on the specimen cell included in the target image as the subject, and
the determination means determines whether it is necessary to correct the parameter, based on a difference between a classification result of each of the plurality of test images selected by the selection means and a classification result of the specimen cell included in the target image as the subject.

### (Supplementary Note C10)

The pathological diagnosis support program according to supplementary note C9, in which the calculation means calculates the similarity, based on a difference between a latent representation obtained by inputting each of the test images to an encoder that is machine learned to output a latent representation of an image, using the image as an input and a latent representation obtained by inputting the target image to the encoder.

### (Supplementary Note C11)

A pathological diagnosis support program for causing a computer to function as a pathological diagnosis support apparatus, the program for causing the computer to function as:
acquisition means for acquiring a target image obtained by capturing, with a camera, an image in which a specimen cell group to be classified as benign or malignant in rapid on-site evaluation is projected with a microscope and a parameter indicating an effective magnification that is a magnification of a size of a subject in the target image, with respect to an actual subject size;
determination means for determining whether it is necessary to correct the parameter;
notification means for notifying that it is necessary to correct the parameter, in a case where the determination means determines that the correction is necessary; and
classification means for performing benign/malignant classification on each specimen cell of the specimen cell group, using the parameter, in a case where the determination means determines that the correction is not necessary.

### [Supplementary Information D]

The present disclosure includes the technologies described in the following Supplementary Notes. However, the present invention is not limited to the technologies described in the following Supplementary Notes, and various modifications can be made within the scope described in the claims.

### (Supplementary Note D1)

A pathological diagnosis support apparatus including at least one processor,
in which the at least one processor executes
acquisition processing for acquiring a target image obtained by capturing, with a camera, an image in which a specimen cell to be classified as benign or malignant in rapid on-site evaluation is projected with a microscope and a parameter indicating an effective magnification that is a magnification of a size of a subject in the target image, with respect to an actual subject size,
determination processing for determining whether it is necessary to correct the parameter,
notification processing for notifying that it is necessary to correct the parameter, in a case where it is determined in the determination processing that the correction is necessary, and
classification processing for performing benign/malignant classification on the specimen cell, using the parameter, in a case where it is determined in the determination processing that the correction is not necessary.

### (Supplementary Note D2)

The pathological diagnosis support apparatus according to supplementary note D1, in which
the at least one processor
further executes correction processing for correcting the parameter, and
in a case where it is determined in the determination processing that the correction is necessary,
corrects the parameter in the correction processing, and
performs benign/malignant classification on the specimen cell, using the parameter corrected in the correction processing, in the classification processing.

### (Supplementary Note D3)

The pathological diagnosis support apparatus according to supplementary note D1 or D2, in which, in the notification processing, the at least one processor includes an indication that a micrometer is to be installed at a viewing angle in the microscope, in an indication that it is necessary to correct the parameter.

### (Supplementary Note D4)

The pathological diagnosis support apparatus according to any one of supplementary notes D1 to D3, in which, in the classification processing, the at least one processor performs benign/malignant classification on the specimen cell included in the target image as the subject, by inputting the target image into a classification model machine learned to classify whether the specimen cell is benign or malignant, using an image including the specimen cell as the subject, as an input.

### (Supplementary Note D5)

The pathological diagnosis support apparatus according to any one of supplementary notes D1 to D4, in which
the at least one processor
further acquires a generation date and time at which the parameter is generated in the acquisition processing, and
determines whether it is necessary to correct the parameter, based on the generation date and time in the determination processing.

### (Supplementary Note D6)

The pathological diagnosis support apparatus according to supplementary note D5, in which
the at least one processor
further acquires a shipping date and time of the camera in the acquisition processing, and
in a case where the generation date and time is the shipping date and time, determines that it is necessary to correct the parameter, in the determination processing.

### (Supplementary Note D7)

The pathological diagnosis support apparatus according to supplementary note D5, in which
the at least one processor
further acquires an attachment/detachment date and time at which the camera is attached/detached, in the acquisition processing, and
in a case where the generation date and time is earlier than the attachment/detachment date and time, determines that it is necessary to correct the parameter, in the determination processing.

### (Supplementary Note D8)

The pathological diagnosis support apparatus according to supplementary note D5, in which, in a case where the generation date and time is earlier than a predetermined date and time, the at least one processor determines that it is necessary to correct the parameter, in the determination processing.

### (Supplementary Note D9)

The pathological diagnosis support apparatus according to any one of supplementary notes D1 to D4, in which
the at least one processor
further executes
calculation processing for calculating a similarity between each of a plurality of test images on which benign/malignant classification is performed in the classification processing and the target image and
selection processing for selecting a predetermined number of test images having the high similarity,
performs benign/malignant classification on the specimen cell included in the target image as the subject, in the classification processing, and
determines, in the determination processing, whether it is necessary to correct the parameter, based on a difference between a classification result of each of the plurality of test images selected in the selection processing and a classification result of the specimen cell included in the target image as the subject.

### (Supplementary Note D10)

The pathological diagnosis support apparatus according to supplementary note D9, in which, in the calculation processing, the at least one processor calculates the similarity, based on a difference between a latent representation obtained by inputting each of the test images to an encoder that is machine learned to output a latent representation of an image, using the image as an input and a latent representation obtained by inputting the target image to the encoder.

### [Supplementary Information E]

The present disclosure includes the technologies described in the following Supplementary Notes. However, the present invention is not limited to the technologies described in the following Supplementary Notes, and various modifications can be made within the scope described in the claims.

### (Supplementary Note E1)

A non-transitory recording medium recording a pathological diagnosis support program which is a program for causing a computer to function as a pathological diagnosis support apparatus, the program causes the computer to execute:
acquisition processing for acquiring a target image obtained by capturing, with a camera, an image in which a specimen cell to be classified as benign or malignant in rapid on-site evaluation is projected with a microscope and a parameter indicating an effective magnification that is a magnification of a size of a subject in the target image, with respect to an actual subject size;
determination processing for determining whether it is necessary to correct the parameter;
notification processing for notifying that it is necessary to correct the parameter, in a case where it is determined in the determination processing that the correction is necessary; and
classification processing for performing benign/malignant classification on the specimen cell, using the parameter, in a case where it is determined in the determination processing that the correction is not necessary. Reference Signs List

- 1, 2: pathological diagnosis support apparatus
- 11: acquisition unit
- 12: determination unit
- 13: notification unit
- 14: classification unit
- 21: correction unit
- 22: calculation unit
- 23: selection unit
- cr: classification result
- le: latent representation
- op: target image
- tp: test image

## Claims

1. A pathological diagnosis support apparatus comprising:
acquisition means for acquiring a target image obtained by capturing, with a camera, an image in which a specimen cell to be classified as benign or malignant in rapid on-site evaluation is projected with a microscope and a parameter indicating an effective magnification that is a magnification of a size of a subject in the target image, with respect to an actual subject size;
determination means for determining whether it is necessary to correct the parameter;
notification means for notifying that it is necessary to correct the parameter, in a case where the determination means determines that the correction is necessary; and
classification means for performing benign/malignant classification on the specimen cell, using the parameter, in a case where the determination means determines that the correction is not necessary.

2. The pathological diagnosis support apparatus according to claim 1, further comprising correction means for correcting the parameter, wherein
in a case where the determination means determines that the correction is necessary,
the correction means corrects the parameter, and
the classification means performs benign/malignant classification on the specimen cell, using the parameter corrected by the correction means.

3. The pathological diagnosis support apparatus according to claim 1 or 2, wherein the notification means includes an indication that a micrometer is to be installed at a viewing angle in the microscope, in an indication that it is necessary to correct the parameter.

4. The pathological diagnosis support apparatus according to any one of claims 1 to 3, wherein the classification means performs benign/malignant classification on the specimen cell included in the target image as the subject, by inputting the target image into a classification model machine learned to classify whether the specimen cell is benign or malignant, using an image including the specimen cell as the subject, as an input.

5. The pathological diagnosis support apparatus according to any one of claims 1 to 4, wherein
the acquisition means further acquires a generation date and time at which the parameter is generated, and
the determination means determines whether it is necessary to correct the parameter, based on the generation date and time.

6. The pathological diagnosis support apparatus according to claim 5, wherein
the acquisition means further acquires a shipping date and time of the camera, and
in a case where the generation date and time is the shipping date and time, the determination means determines that it is necessary to correct the parameter.

7. The pathological diagnosis support apparatus according to claim 5, wherein
the acquisition means further acquires an attachment/detachment date and time at which the camera is attached/detached, and
in a case where the generation date and time is earlier than the attachment/detachment date and time, the determination means determines that it is necessary to correct the parameter.

8. The pathological diagnosis support apparatus according to claim 5, wherein, in a case where the generation date and time is earlier than a predetermined date and time, the determination means determines that it is necessary to correct the parameter.

9. The pathological diagnosis support apparatus according to any one of claims 1 to 4, further comprising:
calculation means for calculating a similarity between each of a plurality of test images on which benign/malignant classification is performed by the classification means and the target image; and
selection means for selecting a predetermined number of test images having the high similarity, wherein
the classification means performs benign/malignant classification on the specimen cell included in the target image as the subject, and
the determination means determines whether it is necessary to correct the parameter, based on a difference between a classification result of each of the plurality of test images selected by the selection means and a classification result of the specimen cell included in the target image as the subject.

10. The pathological diagnosis support apparatus according to claim 9, wherein the calculation means calculates the similarity, based on a difference between a latent representation obtained by inputting each of the test images to an encoder that is machine learned to output a latent representation of an image, using the image as an input and a latent representation obtained by inputting the target image to the encoder.

11. A pathological diagnosis support method performed by at least one processor, comprising:
acquisition processing for acquiring a target image obtained by capturing, with a camera, an image in which a specimen cell to be classified as benign or malignant in rapid on-site evaluation is projected with a microscope and a parameter indicating an effective magnification that is a magnification of a size of a subject in the target image, with respect to an actual subject size;
determination processing for determining whether it is necessary to correct the parameter;
notification processing for notifying that it is necessary to correct the parameter, in a case where it is determined in the determination processing that the correction is necessary; and
classification processing for performing benign/malignant classification on the specimen cell, using the parameter, in a case where it is determined in the determination processing that the correction is not necessary.

12. A program for causing a computer to function as a pathological diagnosis support apparatus, the program causes the computer to execute:
acquisition processing for acquiring a target image obtained by capturing, with a camera, an image in which a specimen cell to be classified as benign or malignant in rapid on-site evaluation is projected with a microscope and a parameter indicating an effective magnification that is a magnification of a size of a subject in the target image, with respect to an actual subject size;
determination processing for determining whether it is necessary to correct the parameter;
notification processing for notifying that it is necessary to correct the parameter, in a case where it is determined in the determination processing that the correction is necessary; and
classification processing for performing benign/malignant classification on the specimen cell, using the parameter, in a case where it is determined in the determination processing that the correction is not necessary.
